# EUROPEAN PATENT APPLICATION

(11) **EP 4 417 266 A1**
(43) Date of publication of application: **21.08.2024**
(21) Application number: 22880800.2
(22) Date of filing: 29.09.2022
(51) Int. Cl.: A61Q 17/04, A61K 8/25, A61K 8/37, A61K 8/89

(54) **COSMETIC COMPOSITION**

(30) Priority: 13.10.2021 JP 2021168433
(71) Applicant: Shiseido Company, Ltd., Chuo-ku Tokyo 104-0061 (JP)
(72) Inventor: NAOI, Kayoko, Tokyo 104-0061 (JP)
(74) Representative: Cabinet Beau de Loménie
(86) International application number: PCT/JP2022/036541
(87) International publication number: WO 2023/063104

(57) **Abstract**

Provided is a cosmetic composition having an ultraviolet absorption effect in the UVA and UVB regions and improved usability.

The cosmetic composition includes an ultraviolet absorber, a hydrophobized silica powder, and an oil content, wherein
the ultraviolet absorber contains a compound I having a structure of formula (I) below: 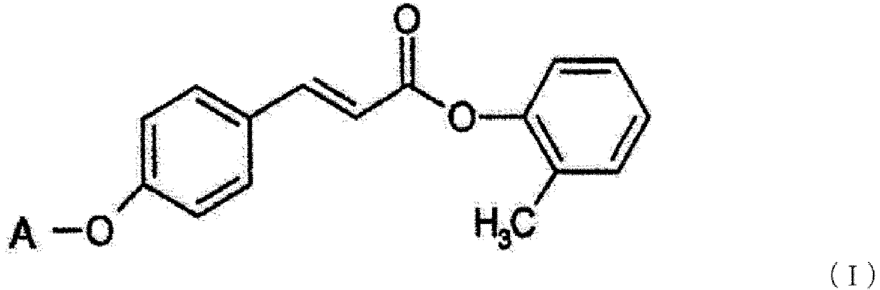 where -OA represents an alkoxy group.

## Description

### FIELD

The present invention relates to a cosmetic composition.

### BACKGROUND

Protecting the skin from ultraviolet rays is an important issue in skin care and body care. In addition to medium-wavelength ultraviolet rays (UVB region: wavelength 290 to 320 nm), which are known to cause sunburn and inflammation, long-wavelength ultraviolet rays (UVA region: wavelength 320 to 400 nm) are known to have an effect on the skin (photoaging, etc.).

Conventional cosmetics for ultraviolet protection often contain 2-ethylhexyl p-methoxycinnamate as an ultraviolet absorber. Though 2-ethylhexyl p-methoxycinnamate has the effect of absorbing ultraviolet rays in the UVB region, there is a need for an ultraviolet absorber having better ultraviolet absorption ability in the UVA region.

In connection thereto, the ultraviolet absorber disclosed in Patent Literature 1 has absorption ability in both the UVA region and the UVB region, has an ultraviolet suppression effect in a wide range of wavelengths, has a previously unseen property: the ultraviolet absorption ability thereof increases in the UVA and UVB regions with the passage of time of ultraviolet irradiation, and has excellent solubility.

More specifically, the ultraviolet absorber of Patent Literature 1 contains, as an active ingredient, a compound represented by general formula I: 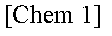 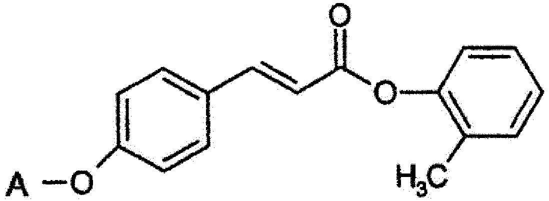 where -OA represents an alkoxy group.

### [CITATION LIST]

### [PATENT LITERATURE]

[PTL 1] WO 2009/041098

### SUMMARY

### [TECHNICAL PROBLEM]

In the present invention, in order to obtain ultraviolet absorption effects in the UVA and UVB regions, cosmetic compositions containing the compound represented by general formula I above, for example, water-in-oil cosmetic compositions, have been investigated.

However, water-in-oil cosmetic compositions containing the compound represented by general formula I above have a problem in that the usability thereof (in particular, usability based on stickiness, resistance to spreading, and ease of skin compatibility) is worse than before blending.

The present invention aims to improve the situation described above, and an object thereof is to provide a cosmetic composition having an ultraviolet absorption effect in the UVA and UVB regions and improved usability.

### [SOLUTION TO PROBLEM]

The present invention, which can achieve the object described above, is as follows.

### <Aspect 1>

A cosmetic composition, comprising an ultraviolet absorber, a hydrophobized silica powder, and an oil content,
wherein the ultraviolet absorber contains a compound I having a structure of formula (I) below: 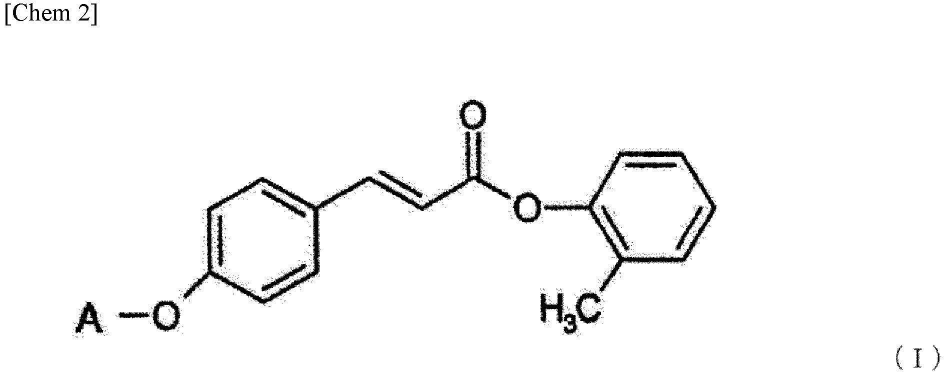 where -OA represents an alkoxy group.

### <Aspect 2>

The composition according to Aspect 1, wherein a content of the compound I is 0.01 to 5.0% by mass.

### <Aspect 3>

The composition according to Aspect 1 or 2,
wherein a content of the ultraviolet absorber is 3.0 to 25% by mass;
wherein a content of the hydrophobized silica powder is 0.5 to 10% by mass; and
wherein a content of the oil content is 5.0 to 60% by mass.

### <Aspect 4>

The composition according to any one of Aspects 1 to 3, wherein the hydrophobized silica powder is a silicone-treated silica powder.

### <Aspect 5>

The composition according to any one of Aspects 1 to 4, wherein the oil content contains a polar oil.

### <Aspect 6>

The composition according to any one of Aspects 1 to 5, wherein the oil content contains a silicone oil.

### <Aspect 7>

The composition according to Aspect 6, wherein the silicone oil contains a volatile acyclic silicone oil.

### <Aspect 8>

The composition according to any one of Aspects 1 to 7, further comprising ultraviolet scattering agent particles.

### <Aspect 9>

The composition according to Aspects 1 to 8, having a viscosity of 500 mPa·s to 10,000 mPa·s, as measured at a temperature of 30 °C and a rotational speed of 12 rpm.

### <Aspect 10>

The composition according to any one of Aspects 1 to 9, which is a water-in-oil composition.

### <Aspect 11>

The composition according to any one of Aspects 1 to 10, which is a two-layer separation-type composition requiring shaking before use.

### <Aspect 12>

The composition according to any one of Aspects 1 to 11, which is a sunscreen cosmetic.

### [ADVANTAGEOUS EFFECTS OF INVENTION]

According to the present invention, a cosmetic composition having an ultraviolet absorption effect in the UVA and UVB regions and improved usability can be provided.

### DESCRIPTION OF EMBODIMENTS

The embodiments of the present invention will be described in detail below. Note that the present invention is not limited to the following embodiments, and various changes can be made within the scope of the spirit of the invention.

### <<Cosmetic Composition>>

The cosmetic composition of the present invention (hereinafter referred to simply as the "composition of the present invention") is:
A cosmetic composition, comprising an ultraviolet absorber, a hydrophobized silica powder, and an oil content,
wherein the ultraviolet absorber contains a compound I having a structure of formula (I) below: 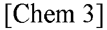 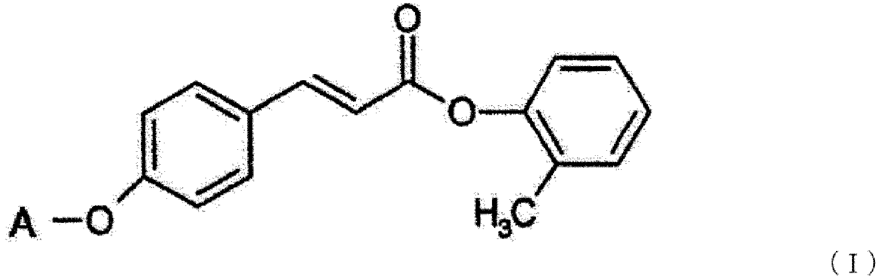
where -OA represents an alkoxy group.

The problem found through rigorous investigation by the present inventors was that when the compound I as an ultraviolet absorber is blended into, for example, a water-in-oil cosmetic composition, the usability of the composition as a whole is reduced.

Conventionally, usability-improving powders for improving the usability of a cosmetic composition, such as silica powder, have been known. However, even when a silica powder was added to a cosmetic composition containing the compound I, no improvement in usability was observed.

Through further rigorous investigation by the present inventors, it was discovered that by using a hydrophobized silica powder in a cosmetic composition containing the compound I, the usability of the composition as a whole was specifically improved.

Note that in the present invention, the usability of a composition refers to the usability based on stickiness, resistance to spreading, and ease of skin compatibility when the composition is applied to the skin. Thus, the composition of the present invention, which has improved usability, is a composition which does not have noticeable stickiness, does not have noticeable resistance to spreading, and has noticeable ease of blending when applied to the skin.

### <Ultraviolet Absorber>

The composition of the present invention comprises an ultraviolet absorber.

In the composition of the present invention, the content of the ultraviolet absorber is not particularly limited, and it may be, for example, 3.0% by mass or more, 4.0% by mass or more, 5.0% by mass or more, 6.0% by mass or more, 7.0% by mass or more, 8.0% by mass or more, 9.0% by mass or more, or 10% by mass or more, and may be 25% by mass or less, 20% by mass or less, 15% by mass or less, or 12% by mass or less.

In the present invention, regarding the content of each component of the composition, unless otherwise specified, the content refers to the content in the entire composition.

### (Compound I)

The ultraviolet absorber according to the present invention comprises the compound I having the structure of the following formula (I): 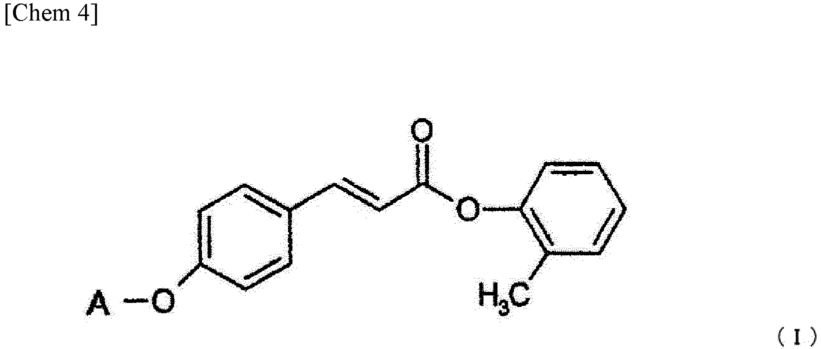 where -OA represents an alkoxy group.

In formula (I), -OA represents an alkoxy group, and more specific examples thereof include, but are not limited to, a methoxy group and an ethoxy group.

In the present invention, the compound I may have the same structure as the compound represented by general formula I disclosed in Patent Literature 1.

By including the compound I, the composition of the present invention has an ultraviolet absorption effect in the UVA and UVB regions.

In the composition of the present invention, the content of the compound I is not particularly limited, and it may be, for example, 0.01% by mass or more, 0.05% by mass or more, 0.1% by mass or more, 0.5% by mass or more, 1.0% by mass or more, 1.5% by mass or more, 2.0% by mass or more, 2.5% by mass or more, or 3.0% by mass or more, and may be 5.0% by mass or less, 4.5% by mass or less, 4.0% by mass or less, 3.5% by mass or less, 3.0% by mass or less, 2.5% by mass or less, or 2.0% by mass or less.

### (Other Ultraviolet Absorber)

In the composition of the present invention, the ultraviolet absorber may further contain an ultraviolet absorber other than the compound I described above.

In the present invention, examples of the other ultraviolet absorbers include, but are not limited to, benzoic acid derivatives, salicylic acid derivatives, cinnamic acid derivatives, dibenzoylmethane derivatives, β,β-diphenylacrylate derivatives, benzophenone derivatives, benzylidene camphor derivatives, phenylbenzimidazole derivatives, triazine derivatives, phenylbenzotriazole derivatives, anthranyl derivatives, imidazoline derivatives, benzalmalonate derivatives, and 4,4-diarylbutadiene derivatives.

Examples of benzoic acid derivatives include, but are not limited to, ethyl para-aminobenzoate (PABA), ethyl-dihydroxypropyl PABA, ethylhexyl-dimethyl PABA, glyceryl PABA, PEG-25-PABA, and diethylaminohydroxybenzoylhexyl benzoate.

Examples of salicylic acid derivatives include, but are not limited to, homosalate, ethylhexyl salicylate, dipropylene glycol salicylate, and TEA salicylate.

Examples of cinnamic acid derivatives include, but are not limited to, octyl methoxycinnamate, isopropyl methoxycinnamate, isoamyl methoxycinnamate, cinnoxate, DEA methoxycinnamate, diisopropyl methylcinnamate, glyceryl-ethylhexanoate-dimethoxycinnamate, and di-(2-ethylhexyl)-4'-methoxybenzalmalonate.

Examples of dibenzoylmethane derivatives include, but are not limited to, 4-tert-butyl-4' -m ethoxydib enzoylm ethane.

Examples of β,β-diphenylacrylate derivatives include, but are not limited to, octocrylene.

Examples of benzophenone derivatives include, but are not limited to, benzophenone-1, benzophenone-2, benzophenone-3 or oxybenzone, benzophenone-4, benzophenone-5, benzophenone-6, benzophenone-8, benzophenone-9, and benzophenone-12.

Examples of benzylidene camphor derivatives include, but are not limited to, 3-benzylidene camphor, 4-methylbenzylidene camphor, benzylidene camphor sulfonic acid, camphor benzalkonium methsulfate, terephthalylidene dicamphor sulfonic acid, and polyacrylamide methylbenzylidene camphor.

Examples of phenylbenzimidazole derivatives include, but are not limited to, phenylbenzimidazole sulfonic acid and disodium phenyl dibenzimidazole tetrasulfonate.

Examples of triazine derivatives include, but are not limited to, anisotriazine, ethylhexyltriazone, diethylhexylbutamide triazone, and 2,4,6-tris(diisobutyl-4'-aminobenzalmalonate)-s-triazine.

Examples of phenylbenzotriazole derivatives include, but are not limited to, drometrizole trisiloxane and methylenebis(benzotriazolyltetramethylbutylphenol).

Examples of anthranyl derivatives include, but are not limited to, menthyl anthranilate.

Examples of imidazoline derivatives include, but are not limited to, ethylhexyldimethoxybenzylidene dioxoimidazoline propionate.

Examples of benzalmalonate derivatives include, but are not limited to, polyorganosiloxanes having benzalmalonate functional groups.

Examples of 4,4-diarylbutadiene derivatives include, but are not limited to, 1,1-dicarboxy(2,2' -dimethylpropyl)-4,4-diphenylbutadiene.

In the composition of the present invention, when another ultraviolet absorber is further included, the content thereof is not particularly limited, and it may be, for example, 1.0% by mass or more, 2.0% by mass or more, 2.5% by mass or more, 5.0% by mass or more, or 10% by mass or more, and may be 20% by mass or less, 15% by mass or less, or 12% by mass or less.

### <Hydrophobized Silica Powder>

The composition of the present invention comprises a hydrophobized silica powder. By including a hydrophobized silica powder, the usability of the composition as a whole can be improved.

In the present invention, "hydrophobized silica powder" refers to silica powder having a surface which has been subjected to a hydrophobization treatment.

In the present invention, the method for the hydrophobization treatment is not particularly limited, and examples thereof include, but are not limited to, a silicone treatment, fatty acid treatment, fatty acid soap treatment, fatty acid ester treatment, higher alcohol treatment, silylation treatment based on a silica aerogel, which is a porous material obtained by replacing the liquid component of silica gel with air (by drying); a method of hydrophobizing and drying a wet silica sol to obtain hydrophobized silica particles, a method of adding a silazane compound or a monofunctional silane compound to a hydrophilic silica particle dispersion to triorganosilylate the silica particle surface to thereby obtain hydrophobized silica particles; a method of hydrolyzing and condensing a tetrafunctional silane compound to obtain a hydrophilic silica particle dispersion, then replacing the hydrophilic organic solvent with water, followed by hydrophobizing with a trifunctional silane compound, and then further replacing the dispersion medium with a ketone solvent to triorganosilylate the reactive groups remaining on the surface of the silica particles with a silazane compound or a monofunctional silane compound to perform hydrophobization treatment, and a method of adding a trifunctional silane compound to a hydrophilic silica particle dispersion to make it hydrophobic, and then adding a monofunctional silane compound to obtain hydrophobized silica particles.

More specifically, examples of the silicone treatment include, but are not limited to, treatment with a silicone oil such as methylhydrogenpolysiloxane, dimethylpolysiloxane, or methylphenylpolysiloxane; an alkylsilane such as methyltrimethoxysilane, ethyltrimethoxysilane, hexyltrimethoxysilane, or octyltrimethoxysilane; or a fluoroalkylsilane such as trifluoromethylethyltrimethoxysilane or heptadecafluorodecyltrimethoxysilane.

Examples of the fatty acid treatment include, but are not limited to, treatment with palmitic acid, isostearic acid, stearic acid, lauric acid, myristic acid, behenic acid, oleic acid, rosin acid, or 12-hydroxystearic acid.

Examples of the fatty acid soap treatment include, but are not limited to, treatments with aluminum stearate, calcium stearate, or 12-hydroxystearic acid.

Examples of the fatty acid ester treatment include, but are not limited to, treatments with a dextrin fatty acid ester, cholesterol fatty acid ester, sucrose fatty acid ester, or starch fatty acid ester.

Furthermore, as the silylation treatment, for example, the silylated silica sold by Dow Coming under the name VM-2270 can be used, but is not limited thereto.

In the present invention, the particle size of the hydrophobized silica powder is not particularly limited, and it may be, for example, 0.01 to 20 µm. Note that the particle size can be evaluated as a particle size of the equivalent area circle when observed with a transmission electron microscope.

In the present invention, one or two or more types of hydrophobized silica powder can be used. In the composition of the present invention, the content of the hydrophobized silica powder is not particularly limited, and it may be, for example, 0.5% by mass or more, 1.0% by mass or more, 1.5% by mass or more, 2.0% by mass or more, 2.5% by mass or more, 3.0% by mass or more, 3.5% by mass or more, 4.0% by mass or more, 4.5% by mass or more, 5.0% by mass or more, 5.5% by mass or more, or 6.0% by mass or more, and it may be 10% by mass or less, 9.5% by mass or less, 9.0% by mass or less, 8.5% by mass or less, 8.0% by mass or less, 7.5% by mass or less, 7.0% by mass or less, 6.5% by mass or less, or 6.0% by mass or less.

### <Oil Content>

The composition of the present invention comprises an oil content.

In the composition of the present invention, the content of the oil content is not particularly limited, and it may be, for example, 5.0% by mass or more, 10% by mass or more, 15% by mass or more, 20% by mass or more, 25% by mass or more, 30% by mass or more, 35% by mass or more, 40% by mass or more, or 50% by mass or more, and may be 60% by mass or less, 55% by mass or less, 50% by mass or less, 45% by mass or less, 40% by mass or less, 35% by mass or less, or 30% by mass or less.

In the present invention, the oil content is not particularly limited, and may include, for example, a polar oil, silicone oil, or hydrocarbon oil. From the viewpoint of dissolution of the compound I described above, the composition of the present invention preferably contains a polar oil. Furthermore, from the viewpoint of suppressing stickiness and the like, the composition of the present invention preferably contains a silicone oil.

### (Polar Oil)

"Polar oil" as used herein refers to polar oils with high polarity among oils usable in cosmetics, other than silicone oils, and refers to those having an IOB value of, for example, 0.10 or more, 0.11 or more, 0.12 or more, or 0.13 or more. Furthermore, the IOB value of the polar oil according to the present invention may be 0.50 or less, 0.45 or less, or 0.40 or less. The IOB value is an abbreviation of Inorganic/Organic Balance, is a value representing the ratio of the inorganic value to the organic value, and is an index indicating the degree of polarity of the organic compound. Specifically, the IOB value is expressed as IOB value = inorganic value / organic value. Regarding the "inorganic value" and the "organic value", depending on the type of atom or functional group, the "inorganic value" or "organic value" is set in a molecule, for example, one carbon atom has an "organic value" of 20 and one hydroxyl group has an "inorganic value" of 100, and by integrating the "inorganic value" or "organic value" of each atom and functional group in an organic compound, the IOB value of the organic compound can be calculated (for example, refer to Yoshio KODA, "Organic Conceptual Diagram - Basics and Applications," pp. 11-17, published by Sankyo Publishing, 1984).

Note that in the present invention, oil contents having an IOB value of 0.10 or more for use as the ultraviolet absorber are excluded from the "polar oils" herein.

In the present invention, the polar oil may be at least one selected from the group consisting of, for example, ester oils, ether oils, higher alcohols, and fatty acids having an IOB value of 0.10 or more.

More specifically, the polar oil may be, for example, at least one selected from the group consisting of bisethoxydiglycol cyclohexane-1,4-dicarboxylate (IOB value 1.03), phenoxyethyl caprylate (IOB value 0.29), PPG-30-BUTETH-30 (IOB value 0.94), diisopropyl sebacate (IOB value 0.4), neopentyl glycol diheptanoate (IOB value 0.33), diethylhexyl succinate (IOB value 0.32), isononyl isononanoate (IOB value 0.2), isopropyl myristate (IOB value = 0.18), octyl palmitate (IOB value = 0.13), isopropyl palmitate (IOB value = 0.16), butyl stearate (IOB value = 0.14), hexyl laurate (IOB value = 0.17), myristyl myristate (IOB value = 0.11), decyl oleate (IOB value = 0.11), isotridecyl isononanoate (IOB value = 0.15), cetyl ethylhexanoate (IOB value = 0.13), pentaerythrityl tetraethylhexanoate (IOB value = 0.35), dioctyl succinate (IOB value = 0.36), glycol distearate (IOB value = 0.16), glyceryl diisostearate (IOB value = 0.29), neopentyl glycol dicaprate (IOB value = 0.25), diisostearyl malate (IOB value = 0.28), trimethylolpropane triisostearate (IOB value = 0.16), glyceryl tri-2-ethylhexanoate (triethylhexanoin) (IOB value = 0.35), trimethylolpropane trioctanoate (IOB value = 0.33), trimethylolpropane triisostearate (IOB value = 0.16), diisobutyl adipate (IOB value = 0.46), N-lauroyl-L-glutamic acid-2-octyldodecyl ester (IOB value = 0.29), 2-hexyldecyl adipate (IOB value = 0.16), ethylhexyl methoxycinnamate (IOB value = 0.28), 2-ethylhexyl palmitate (IOB value = 0.13), 2-ethylhexyl ethylhexanoate (IOB value = 0.2), triisostearin (IOB value = 0.16), PPG-3 dipivalate (IOB value = 0.52), and tri (caprylic acid/capric acid) glyceryl (IOB value = 0.33), but it is not limited thereto.

In the present invention, the amount of the polar oil contained in the oil content is not particularly limited, and it may be, relative to a total of 100 parts by mass of the oil content, for example, 5.0 parts by mass or more, 10 parts by mass or more, 15 parts by mass or more, or 20 parts by mass or more, and may be 90 parts by mass or less, 80 parts by mass or less, 70 parts by mass or less, 60 parts by mass or less, 50 parts by mass or less, 40 parts by mass or less, 30 parts by mass or less, or 20 parts by mass or less.

### (Silicone Oil)

In the present invention, silicone oil refers to oil contents which can be used in cosmetics and which have a main skeleton formed by siloxane bonds.

In the present invention, the silicone oil may be a volatile silicone oil or a nonvolatile silicone oil. From the viewpoint of usability and stability, the silicone oil is preferably a volatile silicone oil.

In the present invention, the boiling point under one atmosphere (101.325 kPa) can be used as a guideline for "volatility." The boiling point may be, for example, 250 °C or lower, 240 °C or lower, or 230 °C or lower, and may be 80 °C or higher, 100 °C or higher, 120 °C or higher, 150 °C or higher, or 160 °C or higher. Furthermore, in the present disclosure, "nonvolatile" is intended to mean that the volatile content is 5% or less when a sample is placed in a sufficiently large flat-bottomed dish and allowed to stand at 105 °C for 3 hours.

Furthermore, in the present invention, the silicone oil may be an acyclic silicone oil (i.e., a linear silicone oil) or cyclic silicone oil. From the viewpoint of usability, the silicone oil is preferably an acyclic silicone oil.

Specific examples of the silicone oil include, but are not limited to, acyclic silicone oils such as polydimethylsiloxane (dimethicone), trisiloxane, caprylylmethicone, methylphenylpolysiloxane, and methylhydrogenpolysiloxane, cyclic silicone oils such as octamethylcyclotetrasiloxane, decamethylcyclopentasiloxane, and dodecamethylcyclohexasiloxane, and mixtures of two or more of these. Among these, volatile acyclic silicone oils such as polydimethylsiloxane (dimethicone) and trisiloxane are particularly preferable.

In the present invention, the amount of the silicone oil contained in the oil content is not particularly limited, and may be, relative to a total of 100 parts by mass of the oil content, for example, 5.0 parts by mass or more, 10 parts by mass or more, 15 parts by mass or more, or 20 parts by mass or more, and may be 90 parts by mass or less, 80 parts by mass or less, 70 parts by mass or less, 60 parts by mass or less, 50 parts by mass or less, 40 parts by mass or less, 30 parts by mass or less, or 20 parts by mass or less.

### (Hydrocarbon Oil)

In the present invention, "hydrocarbon oil" refers to a hydrocarbon oil other than the polar oil described above.

The hydrocarbon oil may be a volatile hydrocarbon oil or a nonvolatile hydrocarbon oil.

Specific examples of the hydrocarbon oil include, but are not limited to, decane, dodecane, isododecane, isohexadecane, liquid paraffin, squalane, squalene, paraffin, and mixtures of two or more thereof.

### <Other Components>

The composition of the present invention may further contain, in addition to the components described above, other components that can be used in the cosmetics field. As the other components, for example, other components such as arbitrary aqueous components, ultraviolet scattering agent particles, surfactants, water-soluble components, moisturizers, sequestering agents, natural and synthetic polymers, water-soluble and oil-soluble polymers, various extracts, colorants such as organic dyes, preservatives, antioxidants, pigments, thickeners, pH adjusters, fragrances, cooling agents, antiperspirants, disinfectants, skin activators, other chemicals, and various powders may be further contained, but the other components are not limited thereto.

### (Aqueous Component)

The composition of the present invention may comprise water as an aqueous component. The water is not particularly limited, and may be ion exchange water.

In the composition of the present invention, when an aqueous component is included, the content thereof is not particularly limited, and it may be, for example, 5.0% by mass or more, 10% by mass or more, 12% by mass or more, 15% by mass or more, 18% by mass or more, 20% by mass or more, 22% by mass or more, 25% by mass or more, 28% by mass or more, 30% by mass or more, 32% by mass or more, 35% by mass or more, 38% by mass or more, 40% by mass or more, 42% by mass or more, or 45% by mass or more, and may be 70% by mass or less, 60% by mass or less, or 50% by mass or less.

### (Ultraviolet Scattering Agent Particles)

The composition of the present invention preferably further comprises ultraviolet scattering agent particles from the viewpoint of enhancing the ultraviolet protection effect.

The ultraviolet scattering agent particles are not particularly limited, and may be, for example, at least one selected from the group consisting of titanium oxide, zinc oxide, iron oxide, and cerium oxide.

The ultraviolet scattering agent particles may be subjected to hydrophobization treatment. The hydrophobization treatment may be performed using a known surface treatment agent for hydrophobization, and examples thereof include a fatty acid treatment, fatty acid ester treatment, fatty acid soap treatment, fluorine compound treatment, silicone treatment, silicone resin treatment, pendant treatment, silane coupling agent treatment, titanium coupling agent treatment, oil treatment, N-acylated lysine treatment, polyacrylic acid treatment, metal soap treatment, amino acid treatment, inorganic compound treatment, plasma treatment, mechanochemical treatment, silane compound treatment, and silazane compound treatment.

The average primary particle diameter of the ultraviolet scattering agent particles is not particularly limited, and may be, for example, 5 nm or more, 10 nm or more, or 15 nm or more, and may be 200 nm or less, 100 nm or less, or 50 nm or less. Note that the "average primary particle diameter" may be determined as the circle equivalent diameter of the projected area of the primary particles in an SEM image.

The shape of the ultraviolet scattering agent particles is not particularly limited, and examples thereof include spherical, plate-like, rod-like, spindle-like, needle-like, and irregular shapes.

In the composition of the present invention, when ultraviolet scattering agent particles are included, the content thereof is not particularly limited, and is, for example, 1.0% by mass or more and 15% by mass or less.

### (Surfactant)

In the present invention, the surfactant may function as a dispersant or an emulsifier, or may have both of these functions.

The surfactant is not particularly limited, and examples thereof include, but are not limited to, isostearic acid, PEG-10 dimethicone, PEG-9 polydimethylsiloxyethyl dimethicone, and lauryl PEG-9 polydimethylsiloxyethyl dimethicone.

In the composition of the present invention, when a surfactant is included, the content thereof is not particularly limited, and is, for example, 0.5% by mass or more and 10% by mass or less.

### (Water-Soluble Component)

The composition of the invention may further comprise a water-soluble component such as a lower alcohol. Examples of the lower alcohol include, but are not limited to, ethanol, propanol, butanol, pentanol, and hexanol.

Further, when a water-soluble component is included, the content thereof is not particularly limited, and may be, for example, 1.0 to 20% by mass.

### <Viscosity of Composition of Present Invention>

From the viewpoint of suppressing dripping during application, the viscosity of the composition of the present invention, as measured at a temperature of 30 °C and a rotational speed of 12 rpm, may be 500 mPa·s or more, 800 mPa·s or more, 1,000 mPa·s or more, 1,500 mPa·s or more, or 2,000 mPa·s or more, and from the viewpoint of improving resistance to spreading and ease of application, may be 10,000 mPa·s or less, 8,000 mPa·s or less, 5,000 mPa·s or less, or 3,000 mPa·s or less.

### <Form of Composition of Present Invention>

The form of the composition of the present invention is not particularly limited, and may be, for example, cream-like, emulsion-like, liquid-like, or powder-like. The composition of the present invention may also be a water-in-oil composition. A water-in-oil cosmetic composition is preferably used from the viewpoint of moisture retention and water resistance.

Furthermore, the composition of the present invention may be, in particular, of a two-layer separation type consisting of two layers separated when left standing and requiring shaking before use.

### <Applications of Composition of Present Invention>

The composition of the present invention can be suitably used as a cosmetic or a raw material thereof. Thus, in particular, the present invention provides a sunscreen cosmetic composition.

### <Method for Production of Composition of Present Invention>

The composition of the present invention can be produced based on a conventional cosmetic preparation method. For example, in the case of a water-in-oil cosmetic composition, the composition of the present invention can be produced by separately preparing the aqueous phase component and the oil phase component, and then gradually adding the aqueous phase component into the oil phase, optionally followed by emulsification with an emulsifier or the like.

### EXAMPLES

The present invention will be described in more detail below with reference to Examples, but the present invention is not limited thereto.

### «Examples, Comparative Examples, and Reference Examples»

Based on the compositions in Table 1 below, a water-in-oil composition was prepared for each of the Examples, Comparative Examples, and Reference Examples.

As the compound I, the compound represented by the following structural formula was used:

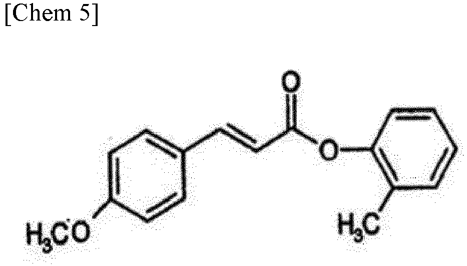

### «Evaluation»

### <Viscosity>

The viscosity of each composition prepared as described above was measured at a temperature of 30 °C and at a rotation speed of 12 rpm using a Type B viscometer with rotor number 3. The results are shown in Table 1.

### <Usability>

The usability of each composition prepared above when applied directly to the face was evaluated based on the following criteria. Note that the evaluation was comprehensively performed by three panelists. The results are shown in Table 1.

### (Stickiness After Application)

"A": No noticeable stickiness;
"B": Slight noticeable stickiness;
"C": Noticeable stickiness.

### (Resistance to Spreading During Application)

"A": Noticeable resistance to spreading;
"B": Slight noticeable resistance to spreading;
"C": Noticeable resistance to spreading.

### (Ease of Skin Compatibility)

"A": Noticeable ease of skin compatibility ;
"B": Slight noticeable lack of ease of skin compatibility ;
"C": No noticeable ease of skin compatibility .

**[Table 1]**

| Composition | | Comparative Example 1 | Example 1 | Comparative Example 2 | Example 2 | Reference Example 1 | Reference Example 2 |
|---|---|---|---|---|---|---|---|
| Water | | Balance | Balance | Balance | Balance | Balance | Balance |
| Alcohol | | 8.0 | 8.0 | 8.0 | 8.0 | 8.0 | 8.0 |
| 1,3-butylene glycol | | 3.0 | 3.0 | 3.0 | 3.0 | 3.0 | 3.0 |
| Glycerin | | 2.0 | 2.0 | 2.0 | 2.0 | 2.0 | 2.0 |
| PEG-9 polydimethylsiloxyethyl dimethic one | | 1.8 | 1.8 | 1.8 | 1.8 | 1.8 | 1.8 |
| PEG-10 dimethicone | | 0.7 | 0.7 | 0.7 | 0.7 | 0.7 | 0.7 |
| Isostearic acid | | 0.5 | 0.5 | 0.5 | 0.5 | 0.5 | 0.5 |
| Disteardimonium hectorite | | 0.5 | 0.5 | 0.5 | 0.5 | 0.5 | 0.5 |
| Dimethicone (oil content) | | 6.0 | 6.0 | 6.0 | 6.0 | 6.0 | 6.0 |
| Caprylyl methicone (oil content) | | 6.0 | 6.0 | 6.0 | 6.0 | 6.0 | 6.0 |
| Trisiloxane (oil content) | | 8.45 | 8.45 | 8.45 | 8.45 | 8.45 | 8.45 |
| Diisopropyl sebacate (oil content) | | 5.0 | 5.0 | 5.0 | 5.0 | 5.0 | 5.0 |
| Trifluoroalkyldimethyltrimethylsiloxysilic ic acid | | 3.0 | 3.0 | 3.0 | 3.0 | 3.0 | 3.0 |
| Octocrylene (UV absorber) | | 3.0 | 3.0 | 3.0 | 3.0 | 3.0 | 3.0 |
| Ethylhexyl methoxycinnamate (UV abs orber) | | 7.5 | 7.5 | 7.5 | 7.5 | 7.5 | 7.5 |
| Compound I (UV absorber) | | 0.01 | 0.01 | 1.0 | 1.0 | - | - |
| Silica powder (untreated) | | 6.0 | - | 6.0 | - | 6.0 | - |
| Dimethicone-treated silica (hydrophobiz ed silica powder) | | - | 6.0 | - | 6.0 | - | 6.0 |
| Dextrin palmitate-treated zinc oxide | | 10 | 10 | 10 | 10 | 10 | 10 |
| EDTA-3Na | | 0.1 | 0.1 | 0.1 | 0.1 | 0.1 | 0.1 |
| Tocopherol | | 0.05 | 0.05 | 0.05 | 0.05 | 0.05 | 0.05 |
| Total (% by mass) | | 100 | 100 | 100 | 100 | 100 | 100 |
| Viscosity (mPa·s) | | 1590 | 2020 | 1680 | 2120 | 1610 | 2310 |
| Usability Evaluation | Stickiness | B | A | C | A | A | A |
| | Resistance to spreading | B | A | C | A | A | A |
| | Ease of skin compatibil ity | B | A | C | A | A | A |

As is clear from Table 1, it was found that there was no difference in usability between the composition of Reference Example 1 (containing untreated silica powder) and the composition of Reference Example 2 (containing hydrophobized silica powder), each of which did not contain the compound I, and both had suitable usability.

Conversely, it was found that the compositions of Comparative Examples 1 and 2, which contained the compound I, had reduced usability as compared to the compositions of Reference Examples 1 and 2, even though untreated silica powder was used.

It was found that the compositions of Examples 1 and 2, which contained the compound I, had improved usability as compared to the compositions of Comparative Examples 1 and 2 due to the inclusion of hydrophobized silica powder, whereby suitable usability could be obtained. From this result, it was suggested that hydrophobized silica powder can specifically improve the usability of compositions containing the compound I.

## Claims

1. A cosmetic composition, comprising an ultraviolet absorber, a hydrophobized silica powder, and an oil content,
wherein the ultraviolet absorber contains a compound I having a structure of formula (I) below: 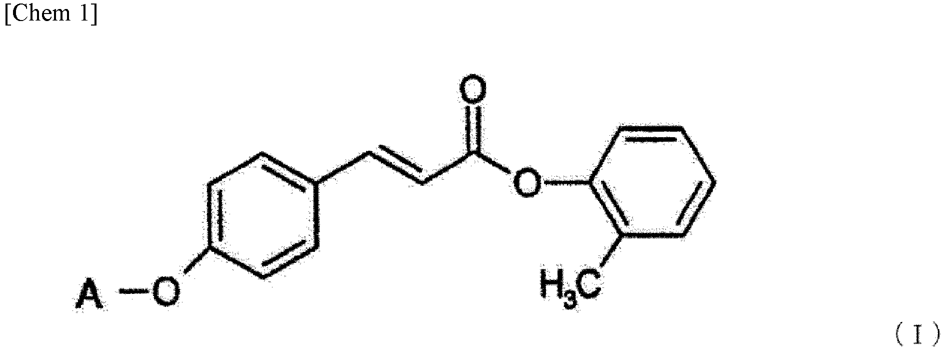 where -OA represents an alkoxy group.

2. The composition according to claim 1, wherein a content of the compound I is 0.01 to 5.0% by mass.

3. The composition according to claim 1 or 2,
wherein a content of the ultraviolet absorber is 3.0 to 25% by mass;
wherein a content of the hydrophobized silica powder is 0.5 to 10% by mass; and
wherein a content of the oil content is 5.0 to 60% by mass.

4. The composition according to any one of claims 1 to 3, wherein the hydrophobized silica powder is a silicone-treated silica powder.

5. The composition according to any one of claims 1 to 4, wherein the oil content contains a polar oil.

6. The composition according to any one of claims 1 to 5, wherein the oil content contains a silicone oil.

7. The composition according to claim 6, wherein the silicone oil contains a volatile acyclic silicone oil.

8. The composition according to any one of claims 1 to 7, further comprising ultraviolet scattering agent particles.

9. The composition according to any one of claims 1 to 8, having a viscosity of 500 mPa·s to 10,000 mPa·s, as measured at a temperature of 30 °C and a rotational speed of 12 rpm.

10. The composition according to any one of claims 1 to 9, which is a water-in-oil composition.

11. The composition according to any one of claims 1 to 10, which is a two-layer separation-type composition requiring shaking before use.

12. The composition according to any one of claims 1 to 11, which is a sunscreen cosmetic.
